# EUROPEAN PATENT SPECIFICATION

(11) **EP 2 632 363 B1**
(45) Date of publication and mention of the grant of the patent: **07.10.2015**
(21) Application number: 11754965.9
(22) Date of filing: 31.08.2011
(51) Int. Cl.: A61B 18/02, A61B 18/14, A61N 1/18, A61N 1/40

(54) **CRYOGENIC-RADIOFREQUENCY ABLATION SYSTEM**
KRYOGENES RADIOFREQUENZABLATIONSSYSTEM
SYSTÈME D'ABLATION CRYOGÉNIQUE PAR RADIOFRÉQUENCE

(30) Priority: 29.10.2010 US 915599
(43) Date of publication of application: 04.09.2013
(73) Proprietor: Medtronic Ablation Frontiers LLC, Minneapolis, MN 55432 (US)
(72) Inventor: LALONDE, Jean-Pierre, Candiac, Québec J5R 3K5 (CA); CONDIE, Catherine R., Shoreview, Minnesota 55126 (US)
(74) Representative: Casey, Lindsay Joseph
(86) International application number: PCT/US2011/049825
(87) International publication number: WO 2012/057915

(56) References cited:
- WO-A1-96/34571
- WO-A2-2005/041748
- WO-A2-2008/049087
- WO-A2-2010/067360
- US-A1- 2002 188 325

## Description

### FIELD OF THE INVENTION

The present invention relates to medical devices and in particular towards medical devices for the thermal treatment of tissue.

### BACKGROUND OF THE INVENTION

Minimally invasive devices, such as catheters, are often employed for medical procedures, including those involving ablation, dilation, and the like. In a particular situation, an ablation procedure may involve creating a series of inter-connecting or otherwise continuous lesions in order to electrically isolate tissue believed to be the source of an arrhythmia. Such lesions may be created using a variety of different energy transmission modalities, such as cryogenic freezing or heating with radiofrequency ("RF") energy

Catheters or devices using cryogenic cooling may be used to lower the temperature of tissue, such as cardiac wall tissue, to an extent such that signal generation or conduction temporarily ceases and allows one to map or confirm that the catheter is positioned at a particular lesion or arrhythmia conduction site. Cryocatheters may also operate at lower temperatures for ablation treatment, e.g., to cool the tissue to a level at which freezing destroys the viability of the tissue, and, in the case of cardiac tissue, permanently removes it as a signal generating or signal conducting locus. Electrically driven RF ablation catheters typically include an arrangement of electrodes configured to contact tissue and apply RF energy thereto so that the tissue heats up due to resistive heating, creating an ablation lesion.

Irrespective of the particular ablation modality employed, the treatment goal common to virtually all cardiac or other ablation treatments is to create an effective lesion and/or provide for the desired, controlled destruction of selected tissues. Whether or not a particular treatment is successful may depend greatly on the qualities or characteristics of the lesion, such as its depth, uniformity, location, or the like. For example, for a given cardiac arrhythmia, a particular lesion depth may be required to effectively obstruct the unwanted signal transmission through the problematic tissue region. RF catheters typically operate quite locally, with the resistive tissue heating declining rapidly with distance from the electrodes. Such limited range of operation may necessitate lengthy treatment procedures involving many iterations of ablative lesion forming, and re-mapping or checking the quality of lesion or symptomatic presence prior to completing a treatment procedure. Such steps may require a lengthy amount of time to perform, thus exposing the patient to undesired risk. Cryogenic lesion creation having sufficient depth may take longer to generate compared to RF treatment, and may require similar repetitions of e-mapping or checking the quality of lesion or symptomatic presence prior to completing a treatment procedure

Accordingly, there remains a need for medical devices that achieve an extended range of thermal transfer while ablating tissue more effectively and to a greater depth.

### SUMMARY OF THE INVENTION

According to an aspect of the present invention, there is provided a medical treatment system as specified in Claim 1. According to another aspect of the present invention, there is provided a medical treatment system as specified in any of claims 2 - 9.

The present invention advantageously provides medical devices that achieve an extended range of thermal transfer while ablating tissue more effectively and to a greater depth. In particular, a medical treatment system is disclosed, including a cooling chamber; a plurality of electrodes spaced across the cooling chamber; a cryogenic fluid source in fluid communication with the cooling chamber; and a radiofrequency signal generator in electrical communication with the plurality of electrodes. The system may include a catheter body, with the cooling chamber located on a distal portion of the catheter body. The catheter body may define a fluid flow path therethrough in fluid communication with the cooling chamber, and the cooling chamber may be defined by an expandable element. Each electrode of the plurality of electrodes may be in the form of a longitudinal strip on the expandable element, and the system may include a console, the console including the cryogenic fluid source and the radiofrequency signal generator.

A medical treatment system is also disclosed, including a catheter body defining a proximal portion, a distal portion, and a fluid flow path therethrough; an expandable element disposed on the catheter body, the expandable element defining a cooling chamber therein in fluid communication with the fluid flow path; a plurality of electrodes disposed on an outer surface of the expandable element; a cryogenic fluid source in fluid communication with the fluid flow path; and a radiofrequency energy source in electrical communication with the plurality of electrodes.

### BRIEF DESCRIPTION OF THE DRAWINGS

A more complete understanding of the present invention, and the attendant advantages and features thereof, will be more readily understood by reference to the following detailed description when considered in conjunction with the accompanying drawings wherein:
FIG. 1 is an illustration of an embodiment of a medical system constructed in accordance with the principles of the present invention;
FIG. 2 is an illustration of an embodiment of a medical device constructed in accordance with the principles of the present invention;
FIG. 3 is an illustration of another embodiment of a medical device constructed in accordance with the principles of the present invention;
FIG. 4 shows a method of use for an embodiment of a medical system constructed in accordance with the principles of the present invention; and
FIG. 5 is a cross-sectional view of an embodiment of a medical device constructed in accordance with the principles of the present invention and a method of use thereof.

### DETAILED DESCRIPTION OF THE INVENTION

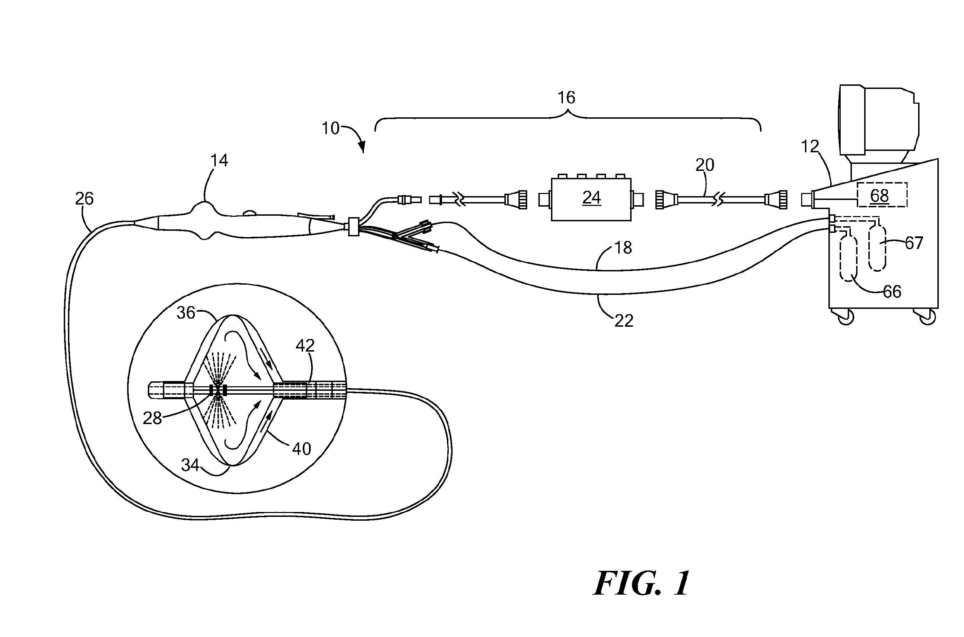

The present invention advantageously provides a medical system to achieve an extended range of thermal transfer while ablating tissue more effectively and to a greater depth. Referring now to the drawing figures in which like reference designations refer to like elements, an embodiment of a medical system constructed in accordance with principles of the present invention is shown in FIG. 1 and generally designated as "10." Of note, the device components have been represented where appropriate by conventional symbols in the drawings, showing only those specific details that are pertinent to understanding the embodiments of the present invention so as not to obscure the disclosure with details that will be readily apparent to those of ordinary skill in the art having the benefit of the description herein. Moreover, while certain embodiments or figures described herein may illustrate features not expressly indicated on other figures or embodiments, it is understood that the features and components of the system and devices disclosed herein are not necessarily exclusive of each other and may be included in a variety of different combinations or configurations without departing from the scope of the invention.

The system generally includes a control unit or console 12 coupled to a medical device 14 through an umbilical system 16. The medical device 14 may be a medical probe, a catheter, a balloon-catheter, as well as other devices deliverable or otherwise positionable through the vasculature and/or proximate to a tissue region for treatment. In particular, the medical device 14 may include a device operable to thermally treat a selected tissue site, including cardiac tissue. The medical system 10 may also include one or more sensors to monitor the operating parameters throughout the system, including for example, pressure, temperature, flow rates, volume, or the like in the console 12, the umbilical system 16, and/or the medical device 14.

Umbilical system 16 may include three separate umbilicals: a coaxial umbilical 18, an electrical umbilical 20 and a vacuum umbilical 22. Although separate umbilicals are shown, it is contemplated that one or more connections may be included in one or more umbilicals having one or more coaxial or otherwise integrally contained passages or conduits therethrough providing electrical and fluid communication between the medical device 14 and the console 12. An outer vacuum umbilical may be suitable for a medical device having multiple layers or balloons. If the user wishes to perform a radiofrequency ("RF") ablation procedure, radiofrequency energy can be provided to electrodes on the medical device 14 via electrical umbilical 20 to perform an RF ablation technique. Electrical umbilical 20 can include an electrocardiograph ("ECG") box 24 to facilitate a connection from one or more electrodes on the medical device 14 to an ECG monitor (not shown). Coaxial umbilical 18 may include both a cooling injection umbilical and a vacuum umbilical that provide respective inlet and return paths for a refrigerant or coolant used to cool a tissue-treating section of the device 14. The vacuum umbilical 22 may provide a safety conduit allowing excess coolant or gas to escape from the device 14 if the pressure within the medical device 14 exceeds a predefined limit. The vacuum umbilical 22 can also be used to capture and remove air or blood leaking into the outer vacuum system when portions of the device are outside or inside the patient, respectively.

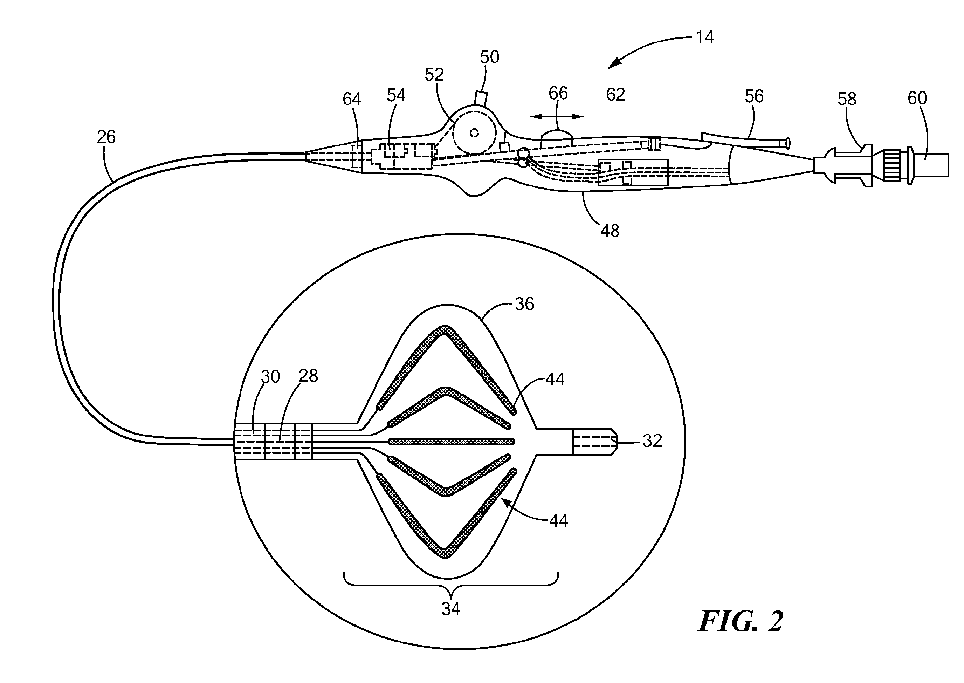

Now referring to FIGS. 1 and 2, the medical device 14 is shown in more detail. The medical device 10 may include an elongate body 26 passable through a patient's vasculature. The elongate body 26 may define a proximal portion and a distal portion, and may further include one or more lumens disposed within the elongate body 26 thereby providing mechanical, electrical, and/or fluid communication between the proximal portion of the elongate body 26 and the distal portion of the elongate body 26. For example, the elongate body 26 may include an injection lumen 28 and an exhaust lumen 30 defining a fluid flow path therethrough. In addition, the elongate body 26 may include a guidewire lumen 32 movably disposed within and/or extending along at least a portion of the length of the elongate body 26 for over-the-wire applications. The guidewire lumen 32 may define a proximal end and a distal end, and the guidewire lumen 32 may be movably disposed within the elongate body 26 such that the distal end of the guidewire lumen 32 extends beyond and out of the distal portion of the elongate body 26.

The medical device may include one or more treatment region(s) 34 for energetic or other therapeutic interaction between the medical device 14 and a treatment site. The treatment regions deliver radiofrequency energy and cryogenic therapy to a tissue area in proximity to the treatment region(s). The device 14 includes a treatment region 34 having a thermal treatment element, being an expandable membrane or balloon and a plurality of electrodes disposed on the elongate catheter body. The treatment region 34 includes a first expandable/inflatable element or balloon 36 defining a proximal end coupled to the distal portion of the elongate body 26 of the medical device 14, while further defining a distal end coupled to the distal end of the guidewire lumen 32. As such, due to the movable nature of the guidewire lumen 32 about the elongate body 26, any axial and/or longitudinal movement of the guidewire lumen 32 may act to tension or loosen the first expandable element 36, i.e., extend or retract the expandable element 36 from a lengthened state to a shortened state during an inflation or deflation thereof. In addition, the first expandable element 36 may have any of a myriad of shapes, and may further include one or more material layers providing for puncture resistance, radiopacity, or the like. The first expandable element 36 is in communication with the fluid injection and exhaust lumens of the medical device 14 as described above. In addition, the fluid injection and/or exhaust lumens may be slidably positionable and movable within the expandable element 36 to direct coolant or fluid dispersion towards a desired portion of the expandable element 36, such as distal or proximal portion.

The medical device 14 may further include a second expandable/inflatable element or balloon 38 contained within or otherwise encompassed by the first expandable element 36 such that an interstitial region, envelope or space 40 is defined therebetween. The second expandable element 38 may define a cooling chamber therein in communication with the fluid injection and exhaust lumens of the medical device 14 as described above, i.e., a first fluid flow path may provide an inflation fluid or coolant, such as a cryogenic fluid or the like, to the interior of the second expandable element 38. Further, the interstitial region 40 may be in fluid communication with an interstitial lumen 42 providing a second fluid flow path or avenue separate and independent from a fluid flow path delivering fluid or otherwise in communication with an interior of the second expandable element 38. The second pathway provides an alternate exhaust route for fluid that may leak from the interior of the second expandable element 38 into the interstitial region 40 or fluid entering the medical device 14 from the exterior. In particular, the isolation of the interstitial lumen 42 from the interior of the second expandable element 38 provides an alternate route for fluid to circulate in the case of a rupture or leak of either the first or second expandable elements, as well as allowing for the injection or circulation of fluids within the interstitial region 40 independently of fluids directed towards the second expandable element 38. Towards that end, the interstitial region may be in fluid communication with a fluid source, a vacuum source, or the like separate from a fluid source, vacuum source or otherwise in fluid communication with the interior of the second expandable element 38. Alternatively, the interstitial lumen 42 may be joined to or otherwise in fluid communication with the injection lumen 28 and the interior of the second expandable element 38 to provide a single exhaust or vacuum source for the medical device 14.

While the first treatment region 34 is in fluid communication with a cryogenic fluid source to cryogenically treat selected tissue, the treatment region 34 also includes a plurality of electrodes coupled to a radiofrequency generator or power source. In particular a plurality of electrodes or electrically conductive portions 46 may be disposed on or otherwise situated about the treatment region 34. For example, as shown in FIG. 2, the electrodes 46 may be deposited or placed onto an exterior surface of the first or second expandable elements such that the electrodes 46 are positionable in proximity to a tissue site for subsequent treatment or diagnostic procedures.

The electrodes are in the form of conductive strips applied to the outer surface of the expandable member, which may be made of metal, conductive polymers, conductive ink printing, or micro-capillary printing. The electrodes may be adhesively bonded to the expandable member or applied by ion-deposition or plasma deposition. The electrodes 44 have the shape of longitudinal strips extending along a length of the expandable element(s) in a proximal-to-distal direction. The electrodes may be arranged at different angular positions around a longitudinal axis of the expandable elements of the medical device. For example, the electrodes may be positioned at discrete locations on the treatment region 34, and may surround or encircle substantially all or only a fractional portion of the expandable members. The electrodes 44 may be asymmetrically disposed about the expandable member. For example, the electrodes may be positioned predominantly towards the proximal or distal portions of the expandable member, and/or on a side of the expandable member likely to face a contacted tissue area.

The electrodes 44 may be customized to provide effective portions selected among a variety of sizes and shapes for contacting or otherwise assessing a tissue treatment area. The size, shape or length of the electrodes 44 may be limited, for example, by covering a portion of the electrode with an insulating material. The dimensions of the electrodes may thus have an optimized configured having sufficient size and a geometric arrangement to effectively treat or diagnose tissue, while avoiding excessive surface area and minimizing reception of 'noise' or other signals. Accordingly, an effective portion of the electrodes is limited to the substantially the entire length/segment of the expandable member 36. Such an arrangement may enable a specific portion of the treatment region 34 to diagnose or treat specific tissues and various shapes of a patient's anatomy.

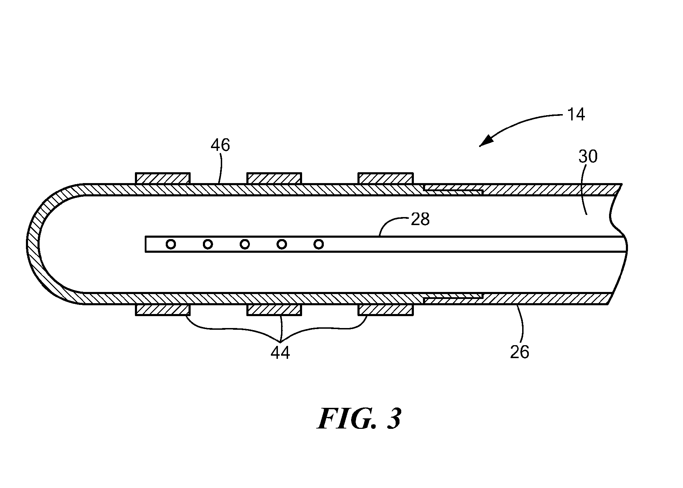

Now referring to FIG. 3, the treatment region 34 of the medical device may include a cooling chamber 46 extending along a length of the distal portion of the elongate body 26 in fluid communication with the fluid lumens described above. The cooling chamber may have a substantially linear configuration, and may also be malleable or otherwise manipulated into other curvilinear and/or convoluted geometric configurations as well. The electrodes 44 may be positioned spaced apart from one another along the length of the cooling chamber 46, and may further circumscribe or otherwise extend around an exterior circumference or surface of the cooling chamber 46 to operate in diagnostic and/or treatment procedures as described in more detail below.

The medical device 14 may further include one or more temperature and/or pressure sensors (not shown) proximate the treatment region(s) for monitoring, recording or otherwise conveying measurements of conditions within the medical device 14 or the ambient environment at the distal portion of the medical device 14. The sensor(s) may be in communication with the console 12 for initiating or triggering one or more alerts or therapeutic delivery modifications during operation of the medical device 14. One or more valves, controllers, or the like may be in communication with the sensor(s) to provide for the controlled dispersion or circulation of fluid through the injection lumens/fluid paths. Such valves, controllers, or the like may be located in a portion of the medical device 14 and/or in the console 12.

Referring to FIG. 2, the medical device 14 may include a handle 48 coupled to the proximal portion of the elongate body 26, where the handle 48 may include an element such as a lever or knob 50 for manipulating the catheter body and/or additional components of the medical device 14. For example, a pull wire 52 with a proximal end and a distal end may have its distal end anchored to the elongate body 26 at or near the distal end. The proximal end of the pull wire 52 may be anchored to an element such as a cam in communication with and responsive to the lever 50. The handle 48 can further include circuitry for identification and/or use in controlling of the medical device 14 or another component of the system. For example, the handle may include one or more pressure sensors 54 to monitor the fluid pressure within the medical device 14. Additionally, the handle may be provided with a fitting 56 for receiving a guidewire that may be passed into the guidewire lumen 32.

The handle 48 may also include connectors that are matable directly to a fluid supply/exhaust and control unit or indirectly by way of one or more umbilicals. For example, the handle may be provided with a first connector 58 that is matable with the co-axial fluid umbilical 18 and a second connector 60 that is matable with the electrical umbilical 20. The handle 48 may further include blood detection circuitry 62 in fluid and/or optical communication with the injection, exhaust and/or interstitial lumens. The handle 48 may also include a pressure relief valve 64 in fluid communication with the injection, exhaust and/or interstitial lumens to automatically open under a predetermined threshold value in the event that value is exceeded.

Continuing to refer to FIG. 2, the medical device 14 may include an actuator element 66 that is movably coupled to the proximal portion of the elongate body 26 and/or the handle 48. The actuator element 66 may further be coupled to the proximal portion of the guidewire lumen 32 such that manipulating the actuator element 66 in a longitudinal direction causes the guidewire lumen 32 to slide towards either of the proximal or distal portions of the elongate body 26. As a portion of either and/or both the first and second expandable elements 36,38 may be coupled to the guidewire lumen 32, manipulation of the actuator element 66 may further cause the expandable element(s) to be tensioned or loosened, depending on the direction of movement of the actuator element 66, and thus, the guidewire lumen 32. Accordingly, the actuator element 66 may be used to provide tension on the expandable element(s) 36,38 during a particular duration of use of the medical device 14, such as during a deflation sequence, for example. The actuator element 66 may include a thumb-slide, a pushbutton, a rotating lever, or other mechanical structure for providing a movable coupling to the elongate body 26, the handle 48, and/or the guidewire lumen 32. Moreover, the actuator element 66 may be movably coupled to the handle 48 such that the actuator element 66 is movable into individual, distinct positions, and is able to be releasably secured in any one of the distinct positions.

In an exemplary system, a fluid supply 68 including a coolant, cryogenic refrigerant, or the like, an exhaust or scavenging system (not shown) for recovering or venting expended fluid for re-use or disposal, as well as various control mechanisms for the medical system may be housed in the console 12. In addition to providing an exhaust function for the catheter fluid supply, the console 12 may also include pumps, valves, controllers or the like to recover and/or re-circulate fluid delivered to the handle 48, the elongate body 26, and treatment region(s) 34 of the medical device 14. A vacuum pump in the console 12 may create a low-pressure environment in one or more conduits within the medical device 14 so that fluid is drawn into the conduit(s) of the elongate body 26, away from the treatment region(s) 34 and towards the proximal end of the elongate body 26. The console 12 may also include a radiofrequency signal generator or power source 68 in electrical communication with the electrodes 44. The console 12 may include one or more controllers, processors, and/or software modules containing instructions or algorithms to provide for the automated operation and performance of the features, sequences, or procedures described herein.

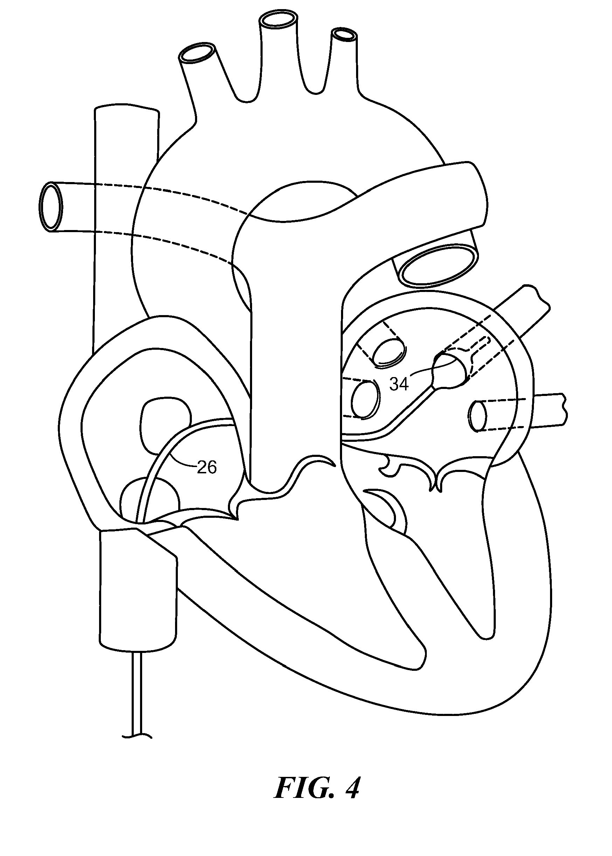

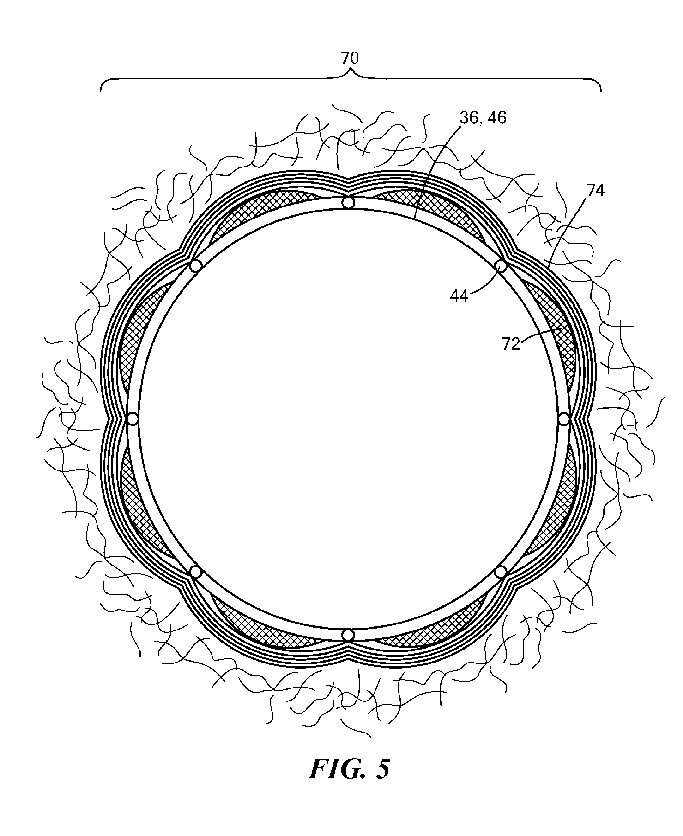

Now referring to FIGS. 4-5, in an exemplary method of use, the medical system 10 may be used to deliver therapeutic treatment to a targeted tissue area 70, which may include a targeted tissue region in the heart. The treatment region 34 may be positioned in the proximity of an opening or orifice in the targeted tissue area, such as a pulmonary vein opening or junction with a portion of the atrial wall, for example. Such positioning may be aided or facilitated by visualization methods including fluoroscopy or the like as known in the art. Where the treatment region 34 includes an expandable element, the expandable element may be inflated or otherwise expanded to substantially occlude the pulmonary vein and place the electrodes 44 adjacent to or otherwise in proximity to the targeted tissue area 70 for subsequent energetic or thermal exchange. The occlusion reduces the blood flow around the treatment region 34, thereby allowing enhanced thermal exchange between the components of the medical device 14 and the targeted tissue.

Once the treatment region 34 is positioned in the desired location, the system 10 may be operated to thermally affect the targeted tissue 70. In particular, the treatment region may synergistically deliver both cryogenic and radiofrequency energy treatment to the targeted tissue to achieve the desired therapeutic effect, such as the controlled ablation of problematic tissue to an effective depth within the targeted tissue region. For example, a cryogenic coolant may be circulated through the treatment region 34 (e.g., through an interior of the expandable elements 36, 38 or through the cooling chamber 46). The cryogenic cooling of the treatment region 34 results in thermal exchange with the surrounding tissue to create frozen tissue regions 72.

During the cooling of the treatment region 34 and thus portions of the targeted tissue region 70, the electrodes 44 may be powered to prevent the freezing of tissue localized around the individual electrodes 44. Powering of the electrodes 44 may include delivery of a radiofrequency signal or current form the radiofrequency source 68 resulting in a current flow, and thus heating, between one or more of the electrodes 44 either between each other (e.g., bipolar RF delivery) or to a ground/patient electrode (not shown) in unipolar or monopolar operation. The operation of the electrodes 44 and the circulation of coolant through the treatment region 34 may be controllably modulated such that tissue regions in between each of the electrodes 44 freezes while the tissue immediately surrounding or otherwise proximate to each electrode 44 remains unfrozen. The electrodes 44 are powered by the radiofrequency signal/power source 68 such that the electrodes 44 are maintained at a predetermined, selected temperature that prevents localized freezing, with the power delivered to the electrodes increasing and decreasing in response to a measured temperature at or near the electrodes, or based upon predetermined parameters and correlations between the electrode temperature, electrode power delivery, and the cooling power/circulation parameters providing the cooling of the expandable elements or cooling chambers of the treatment region 34.

Circulation of the coolant through the treatment region 34 may continue until a desired amount of tissue, e.g. a volume or depth of the tissue, has been frozen. The extent of the tissue freezing with the medical device 14 may be assessed or ascertained by measuring impedance on or about the medical device 14 and the tissue region 70 (using the electrodes 44 for example), by implementing visualization or imaging modalities distinguishing between froze and non-frozen tissue masses, or by providing coolant circulation under predetermined parameters (such as pressure, flow rate, delivery duration, etc.) that have previously been established as providing the desired effect under experimental or pre-clinical investigation.

Upon achieving the desired tissue freezing depth or extent, the electrodes 44 may be operated to ablate the tissue surrounding the frozen tissue regions 72. For example, the electrodes 44 may be powered to induce a current or energy flow between pairs of the electrodes, often referred to as bipolar RF delivery. Current or energy may also be transmitted or otherwise conducted between one or more of the electrodes and a reference or patient electrode on or in the patient, referred to as unipolar or monopolar delivery. Electrical conduction through the frozen tissue is significantly reduced or altogether eliminated, and accordingly, electrical current paths 74 between the electrodes 44 flow around the frozen tissue regions 72, thus driving the current paths deeper into the targeted tissue area 70. By controllably increasing the cooling rate of the treatment region 34 while also correspondingly adjusting the power delivery to the electrodes 44, increased tissue depths can be frozen, thus driving the current paths 74 even deeper into the target tissue region, resulting in a deeper, potentially more effective tissue lesion or ablation site.

In an alternative operation, the electrodes 44 may be powered ablate or otherwise treat tissue until a preselected temperature or power delivery threshold has been reached. The predefined temperature or power delivery threshold may be selected to ensure that the affected tissue is not charred or otherwise heated to an undesirable degree. Upon reaching the temperature or delivered-energy threshold, circulation of the coolant may be initiated to reduce the temperature of the tissue in the vicinity of the electrodes, while the power to the electrodes is subsequently or simultaneously increased. The RF delivery by the electrodes and the circulation of the cryogenic coolant may both then be increased throughout the duration of the procedure to create a deeper treatment region or zone due to the effects of the ice formation between the electrodes, as described above.

In sum, the above-described system takes advantage of the electrical isolation property of frozen tissue, by freezing the tissue between the electrode pairs and forcing the provided radio frequency energy to travel deeper in the periphery of the frozen tissue and promote deeper tissue destruction and ablation. Using a cryogenic chamber or treatment region equipped with electrodes dispersed over its circumference, the cryogenic and radiofrequency energy/delivery may be increased to freeze the tissue between the electrodes. Thus, the radiofrequency energy cannot travel directly from electrode to the other. Instead, it follows the path of lowest impedance, which is the periphery of the frozen tissue. In counterpart, the heat density generated by the electrodes can exceed the cooling power density of the cryogenic chamber or treatment region to prevent the tissue from freezing at the location of the electrodes. Accordingly, low impedance will be maintained to allow radiofrequency energy to be transferred from one electrode to another electrode following the shortest path where the tissue is not frozen. As the cryogenic cooling and radiofrequency energy are increased in a controlled fashion, the tissue continues to freeze deeper and deeper between electrode pairs, forcing the radiofrequency energy to travel even deeper in the tissue, and resulting in a more efficacious treatment procedure and result.

It will be appreciated by persons skilled in the art that the present invention is not limited to what has been particularly shown and described herein above. In addition, unless mention was made above to the contrary, it should be noted that all of the accompanying drawings are not to scale. A variety of modifications and variations are possible in light of the above teachings without departing from the scope of the invention, which is limited only by the following claims.

## Claims

1. A medical treatment system (10), comprising:
an expandable member (36) including a proximal portion and a distal portion, the expandable member (36) further defining a cooling chamber (46);
a plurality of electrodes (44) spaced across the expandable member (36), the effective portions of said plurality of electrodes (44) being uninsulated and extending from the proximal portion to the distal portion of the expandable member (36), wherein each electrode (44) of the plurality of electrodes is in the form of a longitudal strip on the expandable member (36);
a radiofrequency signal generator (68) in electrical communication with the plurality of electrodes (44),
**characterised by**:
a cryogenic fluid source in fluid communication with the cooling chamber (46); and
a control unit (12) coupled to the cryogenic fluid source and the radiofrequency signal generator (68), wherein the control unit (12) is programmed to modulate operation of the cryogenic fluid source and the radiofrequency signal generator (68) to substantially maintain a predetermined target temperature of the plurality of electrodes (44).

2. The system (10) according to Claim 1, further comprising a catheter body (26), the cooling chamber (46) being located on a distal portion of the catheter body (26).

3. The system (10) according to Claim 2, wherein the catheter body (26) defines a fluid flow path therethrough in fluid communication with the cooling chamber (46).

4. The system (10) according to Claim 1, further comprising a console (12), the console (12) including the cryogenic fluid source and the radiofrequency signal generator (68).

5. The system (10) according to Claim 1, wherein the electrodes (44) are asymmetrically disposed on the cooling chamber (46).

6. The system (10) according to Claim 5, wherein the electrodes (44) are disposed asymmetrically towards a distal portion of the cooling chamber (46).

7. The system (10) according to Claim 5, wherein the electrodes (44) are disposed asymmetrically towards a proximal portion of the cooling chamber (46).

8. The system (10) according to Claim 5, wherein the electrodes (44) are disposed across a fraction of the total circumference of the cooling chamber (46).

9. The system (10) according to Claim 1, wherein the control unit (12) is programmed to measure an impedance between at least two of the plurality of electrodes (44).

## Patentansprüche

1. Medizinisches Behandlungssystem (10), das Folgendes umfasst:
ein expandierbares Element (36) mit einem proximalen Abschnitt und einem distalen Abschnitt, wobei das expandierbare Element (36) ferner eine Kühlkammer (46) definiert;
mehrere Elektroden (44), die über das expandierbare Element (36) beabstandet sind, wobei die effektiven Abschnitte der genannten mehreren Elektroden (44) unisoliert sind und vom proximalen Abschnitt zum distalen Abschnitt des expandierbaren Elements (36) verlaufen, wobei jede Elektrode (44) der mehreren Elektroden (44) in Form eines longitudinalen Bandes auf dem expandierbaren Element (36) vorliegt;
einen Funkfrequenzsignalgenerator (68) in elektrischer Verbindung mit den mehreren Elektroden (44),
**gekennzeichnet durch**
eine Kryofluidquelle in Fluidverbindung mit der Kühlkammer (46), und
eine Steuereinheit (12), die mit der Kryofluidquelle und dem Funkfrequenzsignalgenerator (68) gekoppelt ist, wobei die Steuereinheit (12) zum Modulieren des Betriebs der Kryofluidquelle und des Funkfrequenzsignalgenerators (68) programmiert ist, um im Wesentlichen eine vorbestimmte Zieltemperatur der mehreren Elektroden (44) zu halten.

2. System (10) nach Anspruch 1, das ferner einen Katheterkörper (26) umfasst, wobei sich die Kühlkammer (46) an einem distalen Abschnitt des Katheterkörpers (26) befindet.

3. System (10) nach Anspruch 2, wobei durch den Katheterkörper verlaufend ein Fluidströmungspfad (26) in Fluidverbindung mit der Kühlkammer (46) definiert ist.

4. System (10) nach Anspruch 1, das ferner eine Konsole (12) umfasst, wobei die Konsole (12) die Kryofluidquelle und den Funkfrequenzsignalgenerator (68) aufweist.

5. System (10) nach Anspruch 1, wobei die Elektroden (44) asymmetrisch auf der Kühlkammer (46) angeordnet sind.

6. System (10) nach Anspruch 5, wobei die Elektroden (44) asymmetrisch in Richtung auf einen distalen Abschnitt der Kühlkammer (46) angeordnet sind.

7. System (10) nach Anspruch 5, wobei die Elektroden (44) asymmetrisch in Richtung auf einen proximalen Abschnitt der Kühlkammer (46) angeordnet sind.

8. System (10) nach Anspruch 5, wobei die Elektroden (44) über einen Bruchteil des Gesamtumfangs der Kühlkammer (46) angeordnet sind.

9. System (10) nach Anspruch 1, wobei die Steuereinheit (12) zum Messen einer Impedanz zwischen wenigstens zwei der mehreren Elektroden (44) programmiert ist.

## Revendications

1. Système de traitement médical (10), comportant :
un élément extensible (36) comprenant une partie proximale et une partie distale, l'élément extensible (36) définissant par ailleurs une chambre de refroidissement (46) ;
une pluralité d'électrodes (44) espacées en travers de l'élément extensible (36), les parties efficaces de ladite pluralité d'électrodes (44) étant non isolées et s'étendant depuis la partie proximale jusqu'à la partie distale de l'élément extensible (36), dans lequel chaque électrode (44) de la pluralité d'électrodes (44) est sous la forme d'une bande longitudinale sur l'élément extensible (36) ;
un générateur de signaux de radiofréquence (68) en communication électrique avec la pluralité d'électrodes (44),
**caractérisé par** :
une source de liquide cryogénique en communication fluidique avec la chambre de refroidissement (46) ; et
une unité de commande (12) accouplée à la source de liquide cryogénique et au générateur de signaux de radiofréquence (68), dans lequel l'unité de commande (12) est programmée pour moduler le fonctionnement de la source de liquide cryogénique et du générateur de signaux de radiofréquence (68) pour sensiblement maintenir une température cible prédéterminée de la pluralité d'électrodes (44).

2. Système (10) selon la revendication 1, comportant par ailleurs un corps de cathéter (26), la chambre de refroidissement (46) étant située sur une partie distale du corps de cathéter (26).

3. Système (10) selon la revendication 2, dans lequel le corps de cathéter (26) définit un trajet d'écoulement de fluide au travers de celui-ci en communication fluidique avec la chambre de refroidissement (46).

4. Système (10) selon la revendication 1, comportant par ailleurs une console (12), la console (12) comprenant la source de liquide cryogénique et le générateur de signaux de radiofréquence (68).

5. Système (10) selon la revendication 1, dans lequel les électrodes (44) sont disposées de manière asymétrique sur la chambre de refroidissement (46).

6. Système (10) selon la revendication 5, dans lequel les électrodes (44) sont disposées de manière asymétrique vers une partie distale de la chambre de refroidissement (46).

7. Système (10) selon la revendication 5, dans lequel les électrodes (44) sont disposées de manière asymétrique vers une partie proximale de la chambre de refroidissement (46).

8. Système (10) selon la revendication 5, dans lequel les électrodes (44) sont disposées en travers d'une fraction de l'entière circonférence de la chambre de refroidissement (46).

9. Système (10) selon la revendication 1, dans lequel l'unité de commande (12) est programmée pour mesurer une impédance entre au moins deux de la pluralité d'électrodes (44).
